# EUROPÄISCHE PATENTANMELDUNG

(11) **EP 1 681 291 A1**
(43) Veröffentlichungstag der Anmeldung: **19.07.2006**
(21) Anmeldenummer: 06008743.4
(22) Anmeldetag: 09.01.2003
(51) Int. Cl.: C07D 491/06, C07D 307/00, C07D 223/00

(54) **Verfahren zur Herstellung von Norgalanthamin, Ihren Isomeren, Salzen und Hydraten**

(30) Priorität: 25.03.2002 AT 4632002
(62) Teilanmeldung aus: 03702175.5
(71) Anmelder: Sanochemia Pharmazeutika Aktiengesellschaft, 1090 Wien (AT)
(72) Erfinder: Treu, Matthias, 1220 Wien (AT); Hirnschall, Manfred, 1160 Wien (AT); Fröhlich, Johannes, 1050 Wien (AT); Czollner, Laszlo, 2490 Ebenfurth (AT); Kälz, Beate, 7035 Steinbrunn (AT); Kälz, Thomas, 7035 Steinbrunn (AT); Kühnhackl, Peter, 1170 Wien (AT); Jordis, Ulrich, 1180 Wien (AT)
(74) Vertreter: Dungler, Karin

(57) **Zusammenfassung**

Die Erfindung betrifft ein im Industriemaßstab durchführbares Verfahren zur Herstellung von Norgalanthamin, Norgalanthaminderivaten und deren Isomeren der allgemeinen Formel A 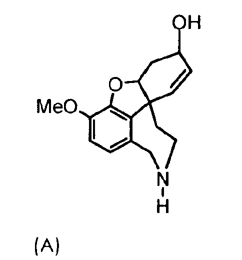 sowie deren Salzen oder Hydraten mit der allgemeinen Formel (B) 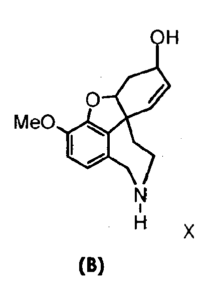 durch katalytische Demethylierung der entsprechenden Galanthaminverbindungen in Gegenwart eines Palladium-Katalysators.

## Beschreibung

Die Erfindung betrifft ein Verfahren zur Herstellung von Norgalanthamin, Norgalanthaminderivaten [= (4aS,6R,8aS)-4a,5,9,10,11,12-hexahydro-3-methoxy-6H-benzofuro [3a,3,2-ef] [2] benzazepin-6-ol, 1 0-De(s) methylgalanthamin, N-Demethylgalanthamin, N-Norgalanthamin] und deren Isomeren mit der allgemeinen Formel A 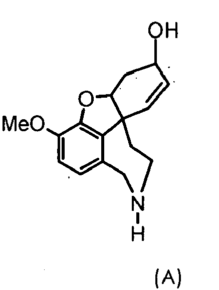 sowie deren Salze und Hydrate mit der allgemeinen Formel B

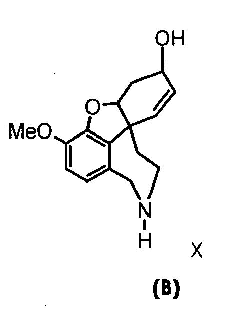

Die Verbindungen, welche unter die allgemeine Formel (A) bzw. (B) fallen, sind Schlüssel-Zwischenprodukte zur Herstellung von synthetischen Galanthaminderivaten, welche u.a. zur Behandlung von Erkrankungen des Zentralnervensystems (ZNS), wie Morbus Alzheimer, verwendet werden.

Verbindungen der allgemeinen Formel (A) und (B) sind bekannt und können beispielsweise aus Naturstoffen gewonnen werden, siehe:
Sener, Bilge; Konukol, Sakine; Kruk, Cornelis, Pandit, Upendra K. **Alkaloids from Amaryllidaceae. III. Alkaloids from the bulbs of Pancratlum maritimum.** Nat. Prod. Sci. 1998,4, 148 - 152
Eichhorn, Jorg; Takada, Takeshi; Kita, Yasuyuki; Zenk, Meinhart H. **Biosynthesis of the Amaryllidaceae alkaloid galanthamine.** Phytochemistry **1998,** 49, 1037 - 1047
Almanza, Giovanna R.; Fernandez, Juan M.; Wakori, Edith W. T.; Viladomat, Francesc; Codina, Carles; Bastida, Jaume. **Alkaloids from Narcissus cv. Salome.** Phytochemistry **1996,** 43, 1375 - 1378
Latvala, Anita; Oenuer, Mustafa A.; Goezler, Tekant; Linden, Anthony; Kivcak, Bijen; Hesse, Manfred. **Alkaloids of Galanthus elwesii.** Phytochemistry **1995**, 39, 1229 - 1240.
Kreh, Mirko; Matusch, Rudolf. **O-Methyloduline** and **N-demethylmasonine, alkaloids from** Narcissus pseudonarcissus. Phytochemistry 1995, 38, 1533 - 1535
Bastida, Jaume; Viladomat, Francesc; Bergonön, Salvador; Fernandez, Juan marcos; Codina, Carles; Rubiralta, Mario; Quirion, Jean Charles. **Narcissus alkaloids. Part 19. Alkaloids from Narcissus leonensis.** Phytochemistry **1993,** 34, 1656 - 1658
Weniger, B.; Italiano, L.; Beck, J.-P.; Bastida, J.; Bergonon, S.; Codina, C.; Lobstein, A.; Anton, R. **Cytotoxic activity of Amaryllidaceae alkaloids.** Planta Med. **1995**, 61, 77 - 79
Bastida, J.; Viladomat, F.; Llabres, J. M.; Quiroga, S.; Codina, C.; Rubiralta, M. Narcissus **alkaloids. Part IX. Narcissus nivalis: a new source of galanthamine.** Planta Med. **1990,** 56, 123- 124
Kihara, Masaru; Koike, Tomomi; Imakura, Yasuhiro; Kida, Kiyoshi; Shingu, Tetsuro; Kobayashi, Shigeru. **Alkaloidal constituents of Hymenocallis rotata Herb. (Amaryllidaceae).** Chem. Pharm. Bull. 1987, 35, 1070 - 1075
Kobayashi, Shigeru; Ishikawa, Hideki; Kihara, Masaru; Shing, Tetsuro; Uyeo, Shojiro. **Isolation of N-demethylgalanthamine from the bulbs of Crinum asiaticum L. var. japanicum Baker (Amaryllidaceae).** Chem. Pharm. Bull. **1976,** 24, 2553 - 2555

Weiters ist es bekannt, Verbindungen der allgemeinen Formel A bzw. B synthetisch durch Demethylierung von Galanthamin herzustellen.

So wird in der WO-A1-9703987 eine zweistufige Demethylierung von Galanthamin durch Abbau des N-Oxids beschrieben.

Weiters wird in Tetrahedron Lett. 1997, 38, 5151, Mary, Aude; Renko, Dolor Zafiarisoa; Guillou, Catherine; Thal, Claude "Selective N-demethylation of galanthamine to norgalanthamine via a non classical Polonovski reaction" eine zweistufige Demethytierung von Galanthamin durch Umsetzen mit Kohlensäurederivaten beschrieben.

Die zweistufige Demethylierung von Galanthamin durch Umsetzen mit Azodicarbonsäureestern und anschließender Verseifung ist aus der US-A-5 958 903 vorbekannt.

Weiters stellt die N-Demethylierung eine klassische Reaktion der Alkaloidchemie dar, wobei insbesondere N-Dealkylierungen mit folgenden Reagentien bekannt sind:
- BrCN/Zn
   Seiler, Max. P.; Hagenbach, Alexander; Wuethrich, Hans-Juerg; Markstein, Rudolf. J. Med. Chem. **1991,** 34, 303 - 307
- Chlorethylchloroformiat
   Ghosh, Debasis; Snyder, Scott E.; Watts, Val J.; Mailman, Richard B.; Nichols, David E. J. Med. Chem. **1996**, 39, 549 - 555
- NaOCl, Phasentransferkatalyse, dann NaOH
   Robson, Claire; Meek, Michelle A.; Grunwaldt, Jan-Dierk; Lambert, Peter A.; Queener, Sherry F. J. Med. Chem. **1997**,*40*, 3040 - 3048
- N-Iodsuccinimid
   Stenmark, Heather G.; Brazzale, Antony; Ma, Zhenkun. J. Org. Chem. **2000,** 65, 3875 - 3876
- Chlorameisensburemethylester
   Kim, J. C. Org. Prep. Proced. Int. **1977,** 9, 1
- Palladium auf Aktivkohle/O₂
   Chaudhuri, Naba K.; Servando, Ofelia; Markus, Bohdan; Galynker, Igor; Sung, Ming-Sang. J. Indian Chem. Soc. **1985,** 62, 899 - 903
- Chlorameisensäureethylester
   Humber, L. G.; Charest, M. P.; Herr, F. J. Med. Chem. **1971,** 14, 982
- Pyridinhydrochlorid
   Radl, Stanislav. **Pyridine hydrochloride In** organic **synthesis.** Janssen Chim. Acta **1989,** 7, 12- 17
- Zahlreiche Chloroformiate
   Olofson, R. A.; Martz, Jonathan T.; Senet, Jean Pierre; Piteau, Marc; Malfroot, Thierry. A new reagent for the selective, high-yield N-dealkylation of tertiary amines: improved syntheses of naltrexone and nalbuphine. J. Org. Chem. 1984, 49, 2081 - 2082
- BrCN/Hydrolyse
   Lee, Shoei Sheng; Liu, Yi Chu; Chang, Shu Hwei; Chen, Chung Hsiung. **N-Demethylation studies of pavine alkaloids.** Heterocycles 1993, 36, 1971 - 1974
- Fe/Fe²⁺/O₂
   Sparfel, Daniel; Baranne-Lafont, Joele; Nguyen Kim Cuong; Capdevielle, Patrice; Maumy, Michel. **II. Catalytic oxidation of 2-(aminomethyl)phenols with the system ferrous chloride-Iron-oxygen.** Tetrahedron **1990**, 46, 803 - 814

Aus der WO-A1-01/74820 ist ein Verfahren zur Herstellung von Norgalanthamin durch Demethylierung in Gegenwart eines Katalysators bekannt. Allerdings wird hier ein relativ teurer Katalysator, nämlich Platin eingesetzt.

Diese bekannten Verfahren haben den Nachteil, dass sie auf Grund ihrer niedrigen Ausbeuten, der relativ teuren Ausgangsprodukte sowie auf Grund der mitunter gefährlichen Reaktionsbedingungen im Industriemaßstab nur schwer durchführbar sind.

Aufgabe der vorliegenden Erfindung ist es daher, Verfahren zum Herstellen von Norgalanthamin, Norgalanthamin-Derivaten sowie deren Isomeren bereitzustellen, welche auch im Industriemaßstab wirtschaftlich durchführbar sind. Gleichzeitig sollen jedoch die Verfahrensendprodukte auch mit hoher Reinheit erzeugt werden.

Die Erfindung betrifft ein Verfahren zur Herstellung von Norgalanthamin, Norgalanthaminderivaten sowie deren Isomeren mit der allgemeinen Formel A bzw. B 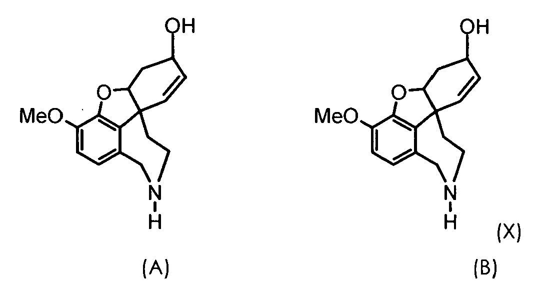 aus den entsprechenden Galanthaminverbindungen (1) 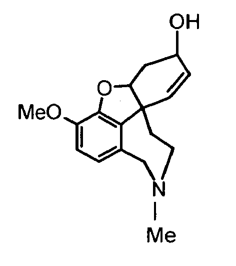 mittels katalytischer Demethylierung unter Verwendung von Palladium als Katalysator. Dabei enthält das Reaktionsgemisch geeignete Lösungsmittel oder Lösungsmittelgemische, welche jedenfalls Methanol enthalten. Der Palladium-Katalysator liegt auf einem geeigneten Trägermaterial, vorzugsweise Calciumcarbonat vor und wird mit einem sauerstoffhältigen Gasgemisch, vorzugsweise Luft, welche an Lösungsmittelgemischen gesättigt ist, behandelt. Weiters kann in besonders vorteilhafter Weise ein Gasgemisch bestehend aus Sauerstoff im Anteil von 3 bis 30 Vol.-% und einem Inertgas wie Stickstoff oder Argon verwendet werden. Anschließend wird der Katalysator aus dem Reaktionsgemisch abgetrennt und das Verfahrensendprodukt mit der allgemeinen Formel B in kristalliner Form, z.B. als Oxalat- oder Hydrochlorid-Salz erhalten. Das erfindungsgemäße Verfahren hat den Vorteil, dass im Gegensatz zu den bekannten Verfahren der Reaktionsverlauf einstufig verläuft und keine gefährlichen oder giftigen Reagenzien eingesetzt werden müssen.

Das erfindungsgemäße Verfahren wird anhand des folgenden Reaktionsschemas sowie eines Ausführungsbeispiels näher erläutert:

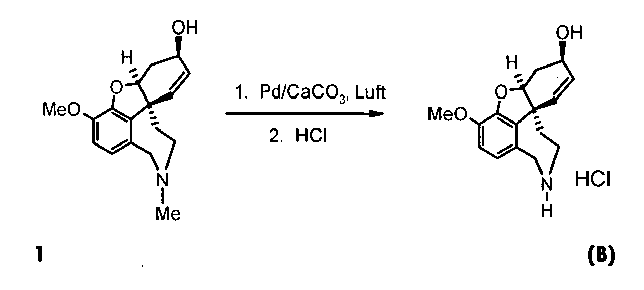

### Beispiel 4:

### Herstellung von (4aS,6R,8aS)-4a,5,9,10,11,12-hexahydro-3-methoxy-6H-Benzofuro(3a,3,2-ef][2]-benzazepin-6-ol, Norgalanthamin Hydrochlorid (B).

10g Galanthamin freie Base, 8g Palladium auf Calciumcarbonat (10 %, Fluka) werden in einem offenen 2 I-Dreihalskolben vorgelegt, 11 über Kaliumcarbonat destilliertes Methanol zugegeben, das Gemisch mit einem Magnetrührer gerührt und mit einem Ölbad auf 50°C erwärmt. Nach sieben-stündigem Rühren wird die Reaktionsmichung gekühlt und der Katalysator absitzen gelassen. Die durch Abdekantieren vom Katalysator befreite Lösung wird am Rotavapor bei 50°C Badtemperatur zur Trockene eingedampft, der Rückstand in 100 ml 2 N Salzsäure gelöst, mit 30 %iger Natronlauge basisch gemacht und 3mal mit je 30 ml Chloroform ausgeschüttelt. Die vereinigten organischen Phasen werden am Rotavapor bei 50°C Badtemperatur bis zur Trockene eingedampft. Der Rückstand wird in 300 ml Ethylacetat aufgenommen, mit 10 %iger etherischer Salzsäure angesäuert und der Niederschlag abfiltriert. Nach einmaligem nachwaschen mit 20 ml Ethylacetat wird das Norgalanthamin Hydrochlorid **(B, X=HCl)** bei 60°C und 25 mbar getrocknet.

Ausbeute 9,4 g (95 % der Th.)
HPLC-Reinheit 87,3 %
Schmp. 166-171°C;

### Beispiel 5:

### Herstellung von (4aS,6R,8aS)-4a,5,9,10,11,12-hexahydro-3-methoxy-6H-Benzofuro[3a,3,2-ef][2]-benzazepin-6-ol, Norgalanthamin (A).

Freisetzen von Norgalanthamin A aus dem Oxalat- oder aus dem Hydrochlorid-Salz:

Das Norgalanthamin-Salz (Oxalat **B,** X=HCl oder Hydrochlorid **B,** X=Oxalsäure) wurde zwischen CH₂Cl₂ und konz. NH₄OH-Lösung verteilt und die wässrige Phase mit CH₂Cl₂ erschöpfend extrahiert. Die vereinigten organischen Phasen wurden getrocknet (Na₂SO₄), filtriert und am Rotationsverdampfer vom Lösungsmittel befreit, wodurch A mit Ausbeuten zwischen 95% und 99% in Form farbloser Kristalle erhalten wurde.

Schmp. 145 - 146 °C; DC: CHCl₃: MeOH : konz. NH₄OH-Lösung = 90 : 8.5 : 1.5; ¹H NMR (CDCl₃): δ 6.65 - 6.52 (m, 2H), 6.06 - 5.92 (m, 2H), 4.57 (b, 1 H), 4.15 - 4.08 (m, 1 H), 3.95 (d, J = 5.7 Hz, 2H), 3.79 (s, 3H), 3.34 (ddd, J = 14.6, J = 3.5 Hz, J = 3.5 Hz, 1H), 3.18 (ddd, J = 13.2, J = 11.4, J = 2.6 Hz, 1 H), 2.66 (ddd, J = 15.7, J = 1.63, J = 1.63 Hz, 1 H), 2.27 (b, 2H), 1.98 (ddd, J = 15.7, J = 5.0, J = 2.4 Hz, 1 H), 1.88 - 1.61 (m, 2H); ¹³C NMR (CDCl₃).: δ 146.2 (s), 143.9 (s), 133.1 (s), 133.0 (s), 127.6 (d), 127.0 (d), 120.5 (d), 111.0 (d), 88.5 (d), 61.9 (d), 55.8 (q), 53.8 (t), 48.7 (s), 47.0 (t), 40.3 (t), 29.9 (t); Anal. ber. für C₁₆H₁₉NO₃: C, 70.31; H, 7.01; N, 5.12. Gefunden: C, 70.05; H, 7.01; N, 4.97.

Zusammenfassend kann gesagt werden, dass mit dem erfindungsgemäßen Verfahren im Industriemaßstab durchführbare Herstellungsmöglichkeiten zum Erzeugen von Norgalanthamin, Norgalanthaminderivaten und deren Isomeren sowie deren Salzen und Hydraten durch katalytische Demethylierung in Gegenwart von Palladium als Katalysator aus den entsprechenden Galanthaminverbindungen angegeben werden.

## Patentansprüche

1. Verfahren zur Herstellung von Norgalanthamin, Norgalanthaminderivaten und deren omeren mit der allgemeinen Formel (A) 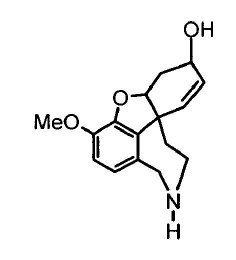 sowie deren Salze oder Hydrate mit der allgemeinen Formel (B) 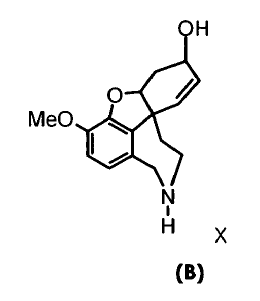 durch katalystische Demethylierung der entsprechenden Galanthaminverbindungen mit der allgemeinen Formel (1) 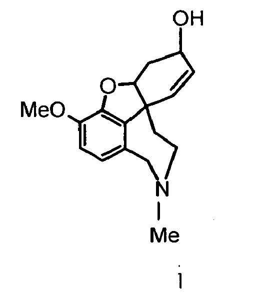 **dadurch gekennzeichnet, dass** als Katalysator Palladium eingesetzt wird.

2. Verfahren nach Anspruch 1, **dadurch gekennzeichnet, dass** die in Gegenwart von Palladium durchgeführte Reaktion eine Einstufenreaktion ist.

3. Verfahren nach Anspruch 1 oder 2, **dadurch gekennzeichnet, dass** der Katalysator mit einem Trägermaterial eingesetzt wird.

4. Verfahren nach Anspruch 3, **dadurch gekennzeichnet, dass** als Trägermaterial Kalziumkarbonat verwendet wird.

5. Verfahren nach einem der Ansprüche 1 bis 4, **dadurch gekennzeichnet, dass** die katalytische Demethylierung in einem zumindest Methonolenthaltenden Lösungsmittelgemisch durchgeführt wird.

6. Verfahren nach einem der Ansprüche 1 bis 5, **dadurch gekennzeichnet, dass** für die Demethylierungs-Reaktion eine Mischung aus Sauerstoff mit einem Inertgas, vorzugsweise Stickstoff oder Argon verwendet wird.

7. Verfahren nach Anspruch 6, **dadurch gekennzeichnet, dass** Sauerstoff in einem Ausmaß von 3 bis 30 Vol. % eingesetzt wird.

8. Verfahren nach Anspruch 6, **dadurch gekennzeichnet, dass** als Inertgas Stickstoff oder Argon verwendet wird.
